# EUROPÄISCHE PATENTSCHRIFT

(11) **EP 0 820 993 B1**
(45) Veröffentlichungstag und Bekanntmachung des Hinweises auf die Patenterteilung: **29.01.2003**
(21) Anmeldenummer: 97111847.6
(22) Anmeldetag: 11.07.1997
(51) Int. Cl.: C07D 241/26

(54) **Verfahren zur Herstellung von Pyrazin-2-aminen**
Process for the production of pyrazin-2-amines
Procédé pour la préparation de pyrazin-2-amines

(30) Priorität: 11.07.1996 CH 173996; 17.10.1996 CH 253996
(43) Veröffentlichungstag der Anmeldung: 28.01.1998
(73) Patentinhaber: Lonza AG, 3930 Visp (CH)
(72) Erfinder: Brieden, Walter, Dr., 3902 Glis (Kanton Wallis) (CH); Fuchs, Rudolf, Dr., 1950 Sion (CH)
(74) Vertreter: Winter, Brandl, Fürniss, Hübner, Röss, Kaiser, Polte Partnerschaft

(56) Entgegenhaltungen:
- E. C. TAYLOR ET AL.: "Pteridines. 48. Utilization of 3,3-Dimethoxy-2-pyrrolidinopropene for the Synthesis of Folic Acid, N(2')-Acetyl-7-folic Acid, and 5-Deaza-7-folic Acid" J. ORG. CHEM., Bd. 46, Nr. 7, 1981, Seiten 1394-1402, XP002049100
- J. B. NEILSEN ET AL.: "Unequivocal Syntheses of 6-Methyl- and 6-Phenylisoxanthopterin" J. HETEROCYCL. CHEM., Bd. 24, Nr. 6, 1987, Seiten 1621-1628, XP002049101
- N. SATO, N. SAITO: "Studies on Pyrazines. 17. An Efficient Synthesis of Pteridine-6-carboxylic Acids" J. HETEROCYCL. CHEM., Bd. 25, Nr. 6, 1988, Seiten 1737-1740, XP002049102
- W. C. LUMMA ET AL.: "Pyridazinylimidazo[1,2-a]pyrazines with Selective Affinity for in Vitro alpha-Adrenergic Receptor Subtypes" J. MED. CHEM., Bd. 26, Nr. 3, 1989, Seiten 357-363, XP002049103
- N. SATO: "Studies on Pyrazines, 8 (1). An Improved Syntheses of 2-Amino-3,5-dibromo- and 2-Amino-5-bromopyrazines" J. HETEROCYCL. CHEM., Bd. 19, Nr. 3, 1982, Seiten 673-674, XP002049104
- F. DENNIN ET AL.: "Synthesis of Derivatives of Pyrazino[1,2-a]pyrimidin-4-ones" J. HETEROCYCL. CHEM., Bd. 27, Nr. 6, 1990, Seiten 1639-1643, XP002049105
- E. ABIGNENTE ET AL.: "Research on heterocyclic compounds. X - Imidazo[1,2-a]pyrazine derivatives: synthesis and antiinflammatory activity" FARMACO ED. SCI., Bd. 36, Nr. 1, 1981, Seiten 61-80, XP002049106
- K. USAMI, M. ISOBE: "Low-temperature Photooxygenation of Coelenterate Luciferin Analog - Synthesis and Proof of 1,2-Dioxetanone as Luminescence Intermediate" TETRAHEDRON, Bd. 52, Nr. 37, 1996, Seiten 12061-12090, XP002049107
- C. F. QUI ET AL.: "Chemi- and Bio-luminescence of Coelenterazine Analogues with Phenyl Homologues at the C-2 Position" J. CHEM. SOC. PERKIN TRANS. 1, Nr. 13, 1992, Seiten 1607-1612, XP002049108
- N. SATO: "Studies on Pyrazines. 4. The Synthesis of 2-Hydroxy-6-phenylpyrazine and its Derivatives" J. HETEROCYCL. CHEM., Bd. 15, Nr. 4, 1978, Seiten 665-670, XP002049109
- E. C. TAYLOR ET AL.: "Pteridines. XXIX. An Unequivoval Route to 2,4-Diamino-6-substituted Pteridines" J. AMER. CHEM. SOC., Bd. 95, Nr. 19, 1973, Seiten 6413-6418, XP002049110
- CHEMICAL ABSTRACTS, vol. 16, no. 24, 1922 Columbus, Ohio, US; G. GASTALDI: "Pyrazines" Seite 101; XP002049189 & GAZZ. CHIM. ITAL., Bd. 51, 1921, Seiten I, 233-255,

## Beschreibung

Die Erfindung betrifft ein neues Verfahren zur Herstellung von 3-Alkoxy-5-alkyl-pyrazin-2-aminen ausgehend entweder von Aminomalonsäuredinitril oder Aminoacetonitril und Glyoxaloximderivaten.

Pyrazin-2-amine sind wichtige Zwischenprodukte zur Herstellung von Pterin-6-carboxyaldehyd (E. C. Taylor & D G. Dumas, J. Org. Chem., 1980, S. 2485).

Die GB-A 922 725 beschreibt ein Verfahren zur Herstellung von 3-Methoxy-5-methyl-pyrazin-2-amin ausgehend von 2-Amino-3-chlor-5-methylpyrazin durch Reaktion von Natriummethanolat bei 130 °C. Nachteilig bei diesem Verfahren ist die relativ lange Umsetzungszeit

Aufgabe der vorliegenden Erfindung war, ein wirtschaftliches Verfahren zur Herstellung von Pyrazin-2-aminen zur Verfügung zu stellen, wobei diese in guter Ausbeute erhalten werden

Diese Aufgabe wird mit dem neuen Verfahren gemäss Anspruch 1 gelöst

Erfindungsgemäss wird das Verfahren in der ersten Stufe derart durchgeführt, dass man Aminomalonsäuredinitril, oder dessen Salz, der Formel 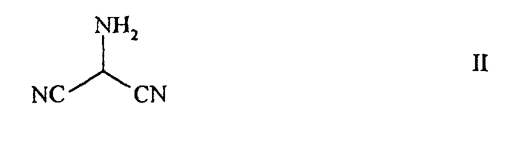 mit einem Glyoxaloximderivat der allgemeinen Formel 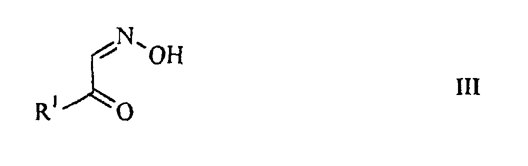 auf an sich bekannte Weise gemäss Taylor et al., (J. Am. Chem Soc., 95(10), 1973, 6413-6418) in ein Oxypyrazincarbonitrilderivat der allgemeinen Formel 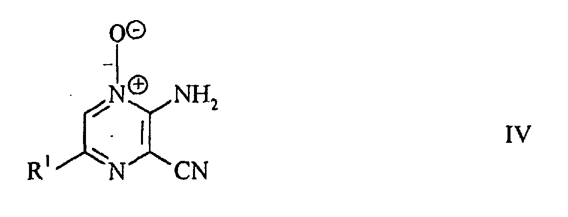 überführt

Die beiden Edukte, das Aminomalonsäurenitril und die Glyoxaloximderivate, sind käufliche Verbindungen. Als Salz des Aminomalonsäurenitrils kann bspw Aminomalonsaurenitriltosylat eingesetzt werden.

Der Rest R¹ bedeutet C₁₋₄-Alkyl oder Aryl. Als C₁₋₄-Alkyl kann z. B Methyl, Ethyl, Propyl, iso-Propyl, Butyl, iso-Butyl oder tert-Butyl eingesetzt werden Als Aryl kann Phenyl oder Benzyl, substituiert oder unsubstituiert, eingesetzt werden. Beispiele für substituiertes Phenyl bzw substituiertes Benzyl sind 4-Methoxyphenyl bzw. 4-Methoxybenzyl

Zweckmässig wird die Umsetzung in der ersten Stufe in einem polaren Lösungsmittel durchgeführt. Als polares Lösungsmittel können Wasser, niedere Carbonsäuren, niedere Alkohole, Nitrile wie Acetonitril, Dimethylformamid, Dimethylacetamid oder Dimethylsulfoxid verwendet werden Als niedere Alkohole können Methanol, Ethanol, Propanol, iso-Propanol, Butanol, iso-Butanol, tert-Butanol eingesetzt werden Als niedere Carbonsäuren können Ameisensäure, Essigsäure, Propionsäure oder Buttersaure verwendet werden

Die Umsetzung in der ersten Stufe wird zweckmässig unter Inertgasatmosphäre und bei einer Temperatur von 0 bis 180 °C, vorzugsweise von 10 bis 30 °C, durchgeführt

In der zweiten Stufe wird das Oxypyrazincarbonitrilderivat (Formel IV) in ein Oxypyrazincarbonsäuresalz der allgemeinen Formel 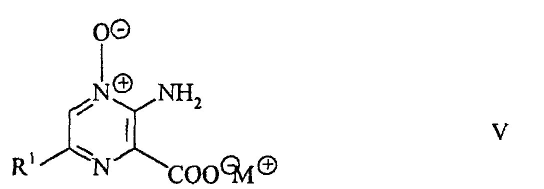 hydrolysiert
Im Oxypyrazincarbonsauresalz bedeutet M ein Metallatom wie z. B. ein Alkalimetallatom, ein Erdalkalimetallatom oder Ammonium. Insbesondere bedeutet M ein Alkalimetallatom.

Zweckmässig wird die Hydrolyse in wässrigem Milieu und in Gegenwart einer Base oder einer Säure durchgeführt. Als Base kann ein Alkali- oder Erdalkalimetallhydroxid oder ein Ammoniumsalz verwendet werden. Als Alkalimetallhydroxid kann Natrium- oder Kaliumhydroxid verwendet werden Als Erdalkalymetallhydroxid kann Calcium- oder Magnesiumhydroxid eingesetzt werden. Als Säure kann Schwefelsäure oder eine Halogenwasserstoffsäure wie HCI, HBr oder HJ verwendet werden. Vorzugsweise wird die Hydrolyse in Gegenwart einer Base durchgefuhrt.

Die Hydrolyse wird zweckmässig unter Inertgasatmosphäre und bei einer Temperatur von 0 bis 100 °C durchgeführt Vorzugsweise wird die Hydrolyse bei einer Temperatur von 50 bis 80 °C durchgeführt.

In der dritten Stufe wird das Oxypyrazincarbonsäuresalz (Formel V) zu einem Halooxypyrazin der allgemeinen Formel 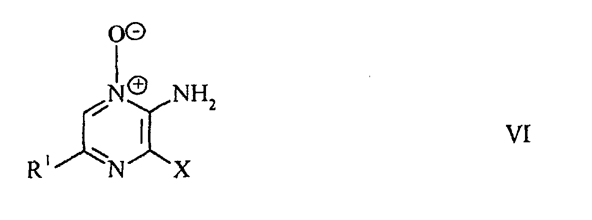 halogeniert

Der Substituent X bedeutet ein Halogenatom wie Fluor, Chlor, Brom oder Jod. Die Halogenierung kann mit einem Halogen oder mit Halogensauerstoffsäuren bzw deren Salze durchgeführt werden.

Als Halogen kann Cl₂, Br₂ oder J₂, vorzugsweise Br_{2.} eingesetzt werden. Als Halogensauerstoffsäuren können folgende eingesetzt werden: HCIO, HBrO, HJO, HClO₃, HBrO₃ oder HJO₃ bzw. deren Alkalimetallsalze wie KOBr oder NaBrO₃.

Zweckmässig wird die Halogenierung in der dritten Stufe bei einer Temperatur von -30 bis 100 °C, vorzugsweise bei einer Temperatur von 0 bis 30 °C, durchgeführt

Geeignete Lösungsmittel für die Halogenierung sind polare Lösungsmittel wie Wasser, niedere Alkohole oder wassrige niedere Alkohole. Als niedere Alkohole können Methanol, Ethanol, Propanol oder Butanol verwendet werden.

In der vierten Stufe wird das Halooxypyrazinamin (Formel VI) entweder mit einem Alkohol in Gegenwart einer Base oder mit einem Alkoholat in ein 1-Oxypyrazin-2-ylamin der allgemeinen Formel 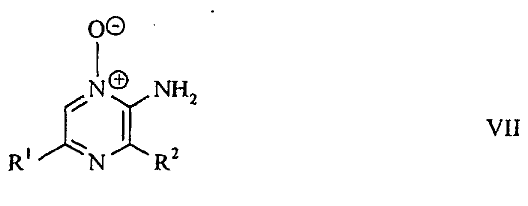 worin R₁ die genannte Bedeutung hat und R² eine C₁₋₄-Alkoxygruppe oder eine Aryloxygruppe bedeutet, überfuhrt.

Als C₁₋₄-Alkoxygruppe kann Methoxy, Ethoxy, Propoxy, iso-Propoxy, Butoxy, iso-Butoxy oder tert-Butoxy eingesetzt werden Als Aryloxy kann Phenyloxy oder Benzyloxy, substituiert oder unsubstituiert, verwendet werden. Als Beispiel für substituiertes Phenyloxy bzw. Benzyloxy kann 4-Methoxyphenyloxy bzw 4-Methoxybenzyloxy angegeben werden.

Als Alkoholate werden zweckmässig die entsprechenden C₁₋₄-Alkalimetallalkoholate oder Arylalkalimetallalkoholate eingesetzt. Als C₁₋₄-Alkalimetallalkoholat kann ein Natrium- oder Kaliummethanolat, Natrium- oder Kaliumethanolat, Natrium- oder Kaliumpropanolat oder Natrium- oder Kaliumbutanolat eingesetzt werden. Als Arylalkalimetallalkoholat kann Natrium- oder Kaliumphenolat oder Natrium- oder Kaliumbenzylalkoholat verwendet werden. Als Alkohole werden zweckmässig die entsprechenden C₁₋₄-Alkohole oder Arylalkohole, wie bspw. Methanol, Ethanol, Propanol, iso-Propanol, Butanol, tert-Butanol, iso-Butanol, Phenol oder Benzylalkohol eingesetzt. Als Basen können die gleichen wie in der zweiten Stufe verwendet werden

In der vierten Stufe wird üblicherweise als Lösungsmittel der entsprechende Alkohol oder der entsprechende wässrige Alkohol eingesetzt

Zweckmassig wird die Umsetzung in der vierten Stufe unter Inertgasatmoshäre und bei einer Temperatur von 0 bis 180 °C, vorzugsweise bei einer Temperatur von 20 bis 80 °C, durchgefuhrt

In der fünften Stufe wird das 1-Oxypyrazin-2-ylamin (Formel VII) ins Endprodukt der allgemeinen Formel I reduziert.

Zweckmässig wird die Reduktion in der fünften Stufe katalytisch mit Wasserstoff, PCl₃ oder Na₂S₂O₄, vorzugsweise katalytisch mit Wasserstoff, durchgeführt.

Als Hydrierkatalysatoren können Edelmetall-, Edelmetalloxid oder Raney-Katalysatoren, gegebenfalls aufgebracht auf einem Trägermaterial, angewendet werden. Beispielsweise können als Hydrierkatalysatoren Raney-Nickel, Platin auf Kohle oder Platin auf Aluminiumoxid verwendet werden. Zweckmassig wird als Hydrierkatalysator Platin auf Kohle, insbesondere 1 bis 10 Gew % Platin auf Kohle, verwendet. Die Hydrierkatalysatoren können in einer Menge von 0,1 bis 40 Gew.%., vorzugsweise von 5 bis 20 Gew % bezogen auf 1-Oxypyrazin-2-ylamin verwendet werden. Zweckmässig erfolgt die Hydrierung bei einem erhöhten H₂-Druck, vorzugsweise bei einem Druck von 1 bis 10 bar

Zweckmässig wird die Reduktion in der fünften Stufe in einem polaren Lösungsmittel durchgeführt. Als polares Lösungsmittel sind beispielsweise niedere Alkohole, Wasser, Dimethylformamid oder Carbonsäuren geeignet Als niedere Alkohole können dieselben wie in der dritten Stufe verwendet werden. Als Carbonsaure kann bspw Ameisensäure oder Essigsaure eingesetzt werden.

Die Reduktion wird zweckmässig unter Inertgasatmosphäre und bei einer Temperatur von 0 bis 250 °C, vorzugsweise von 120 bis 180 °C, durchgeführt

In einer bevorzugten Ausführungsform wird das gesamte Verfahren ohne Isolation des Oxypyrazincarbonsäuresalzes (Formel V) durchgeführt.

In einer weiteren erfindungsgemässen Ausführungsform wird die erste Verfahrensstufe derart durchgeführt, dass man Aminoacetonitril oder dessen Salz der Formel

H₂N-CH₂-CN VIII

mit einem Glyoxaloximderivat der allgemeinen Formel 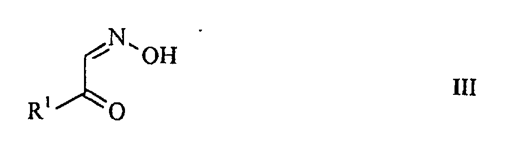 worin R¹ die genannte Bedeutung hat, in Gegenwart einer Base auf an sich bekannte Weise gemäss J Heterocycl Chem, 1985, 1145 - 1146 in ein Oxypyrazinamin der allgemeinen Formel 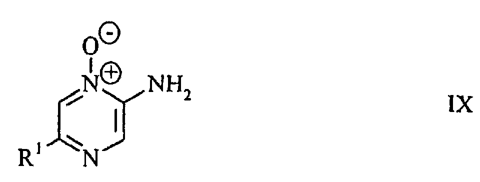 worin R¹ die genannte Bedeutung hat, überführt.

Das Edukt Aminoacetonitril ist käuflich. Als Salze des Aminoacetonitrils können dessen Hydrochlorid- oder Hydrobromid-Salze verwendet werden.

Als Base kann Methylmorpholin, Triethylamin, Pyridin, ein Alkalimetallhydroxid, ein Alkoholat oder ein Alkalimetallcarbonat eingesetzt werden. Als Alkoholate können dieselben wie in der vierten Stufe verwendet werden. Als Alkalimetallhydroxide können dieselben wie in der zweiten Stufe eingesetzt werden. Als Alkalimetallcarbonat kann Natrium- oder Kaliumcarbonat verwendet werden.

Zweckmässig wird gemass dieser Verfahrensvariante die Umsetzung in der ersten Stufe in einem polaren Lösungsmittel durchgeführt. Als polares Lösungsmittel können Halogenkohlenwasserstoffe wie Dichlormethan, Chloroform, Tetrachlormethan, Tri-, Tetrachlorethan, Ethylenchlorid oder Chlorfluorkohlenwasserstoffe, die zuvor beschriebenen niederen Alkohole, die zuvor beschriebenen niederen Carbonsäuren, Wasser, Dimethylformamid oder Dimethylacetamid verwendet werden.

Die Umsetzung in der ersten Stufe wird zweckmässig unter Intergasatmosphäre und bei einer Temperatur von -30 bis 100 °C, vorzugsweise von 0 bis 40 °C, durchgefuhrt

In der zweiten Stufe dieser Verfahrensvariante wird das Oxypyrazinamin (Formel IX) zu einem Halooxypyrazinamin der allgemeinen Formel 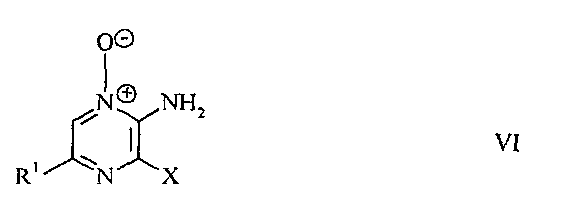 worin R¹ und X die genannte Bedeutung haben, halogeniert.

Als Halogenierungsreagenzien und als Lösungsmittel für die Halogenierung können dieselben wie in der zuvor beschriebenen Variante verwendet werden

Zweckmässig wird die Halogenierung gemäss dieser Variante bei einer Temperatur von -30 bis 100 °C, vorzugsweise bei einer Temperatur von 0 bis 30 °C, durchgeführt.

Die weiteren Reaktionsstufen dieser Verfahrensvariante werden analog zur zuvor beschriebenen Variante durchgeführt. D.h. die dritte Stufe dieser Variante wird analog zur vierten Stufe der zuvor beschriebenen Variante und die vierte Stufe dieser Variante wird analog zur fünften Stufe der zuvor beschriebenen Variante durchgefuhrt. Zweckmässig wird diese Verfahrensvariante ohne Isolation des Zwischenproduktes der Formel VI durchgeführt

Sowohl die Oxypyrazincarbonsäuresalze (Formel V), das Halooxypyrazinamine (Formel VI) als auch das 1-Oxypyrazin-2-ylamin (Formel VII) sind in der Literatur noch nicht beschriebene Verbindungen und damit als neue Zwischenprodukte zur Herstellung von Pyrazin-2-aminen Bestandteil der Erfindung.

Zweckmässig bedeutet M im Oxypyrazincarbonsäuresalz ein Alkalimetallatom wie Natrium oder Kalium

Der Substituent X im Halooxypyrazinamin (Formel VI) hat die bereits beschriebene Bedeutung und bedeutet zweckmässig Brom
Der Rest R¹ im Halooxypyrazinamin (Formel VI) und im 1-Oxypyrazin-2-ylamin (Formel Vll) hat die bereits beschriebene Bedeutung und bedeutet zweckmässig C₁₋₄-Alkyl, vorzugsweise Methyl. Im 1-Oxypyrazin-2-ylamin (Formel VII) hat R² die bereits beschriebene Bedeutung und bedeutet zweckmässig C₁₋₄-Alkoxy, vorzugsweise Methoxy.

Die am meisten bevorzugten Verbindungen sind demzufolge 3-Brom-5-methyl-1-oxopyrazin-2-ylamin (Formel Vl) und 3-Methoxy-5-methyl-1-oxypyrazin-2-ylamin (Formel VII)

### Beispiel 1

### Herstellung von 3-Amino-6-methyl-4-oxypyrazin-2-carbonitril

34,80 g von Anti-methylglyoxal-1-oxim (391,6 mMol) und 98,15 g Aminomalonsauredinitriltoluol-4-sulfonat (379 mMol) wurden mit 600 ml Isopropanol in einem 1000 ml Kolben unter Argon vorgelegt und 4 h bei 20 °C gerührt Nach Abkühlung auf 5 °C, wurde das Produkt filtriert und mit 40 ml kaltem Wasser, 40 ml kaltem Ethanol gewaschen. Nach Trocknung erhielt man ein rohes Produkt (55,28 g). Das rohe Produkt wurde 30 Min, bei 85 °C, in 150 ml Ethanol gerührt (Aufschlämmung). Das Produkt wurde bei 14 °C filtriert und getrocknet. Man erhielt 53,35 g 3-Amino-6-methyl-4-oxypyrazin-2-carbonitril. Ausbeute: 91,3%.

| | |
|---|---|
| ¹H-NMR (DMSO-d₆, 400 MHz) δ | 2,29 (s, 3H); |
| | 7,70 (s, 2H); |
| | 8,45 (s,1H). |

Schmelzpunkt. 187 - 188 °C

### Beispiel 2

### Herstellung von 3-Amino-6-phenyl-4-oxypyrazin-2-carbonitril

25,1 g von lsonitrosoacetophenon (167,4 mMol) und 41,9 g Aminomalonsäuredinitril-toluol-4-sulfonat 162,1 mMol) wurden mit 250 ml Isopropanol in einem 500 ml Kolben unter Argon vorgelegt und 4 h bei 20 °C gerührt Nach Abkühlung auf 5 °C, wurde das Produkt filtriert und mit 40 ml kaltem Wasser, 40 ml kaltem Ethanol gewaschen Nach Trocknung erhielt man ein rohes Produkt (33,12 g) Das rohe Produkt wurde dreimal, bei
20 °C, in 100 ml Wasser gerührt (Aufschlämmung) und dann filtriert. Man erhielt 11 g 3-Amino-6-phenyl-4-oxypyrazin-2-carbonitril,
Ausbeute 32%

| | |
|---|---|
| ¹H-NMR (DMSO-d₆, 400 MHz) δ· | 7,45 (m, 3H); |
| | 7,97 (m, 2H), |
| | 8,05 (s,2H); |
| | 9,18 (s,1H) |
| ¹³C-NMR (DMSO-d₆, 400 MHz) δ· | 149,519 |
| | 142,298 |
| | 134,147 |
| | 131,306 |
| | 129,286 |
| | 128,797 |
| | 125,609 |
| | 115,106 |
| | 11,184 |

### Beispiel 3

### Herstellung von 3-Brom-5-methyl-1-oxopyrazin-2-ylamin ohne Isolation von Zwischenprodukt (Formel V)

Das 3-Amino-6-methyl-4-oxopyrazin-2-carbonitril (10,04 g) (66,8 mMol), KOH (5,23 g-85%ig) (86,3 mMol) und Wasser (66 g) wurden in einem 250 ml Kolben unter Argon vorgelegt und 3 h bei 45 °C aufgewärmt Dann, bei 18 °C, wurde in 30 Minuten 10,6 g Brom (66,3 mMol) zugetropft (Exothermie und Gasentwicklung). Die Temperatur stieg bis 28 °C. Man rührte 1 h bei 20 °C und man gab 40 ml n-Butanol zu für die Extraktion. Nach der Phasetrennung extrahierte man zweimal mit 30 ml n-Butanol. Die organische Phase wurde vollständig eingeengt Nach Trocknung erhielt man 9,4 g 3-Brom-5-methyl-1-oxopyrazin-2-ylamin
Ausbeute 68%

| | |
|---|---|
| ¹H-NMR (DMSO-d₆, 400 MHz) δ. | 2,15 (s, 3H); |
| | 6,90 (s, 2H); |
| | 8,18 (s, 1H) |

### Beispiel 4

### Herstellung von 3-Methoxy-5-methyl-1-oxypyrazin-2-ylamin

a) Unter Argon wurden das 3-Brom-5-methyl-1-oxopyrazin-2-ylamin (3,57 g) (16,8 mmol) und Methanol (50 ml) vorgelegt. Bei 20 °C, tropfte man in 15 Min die Natriummethanolat-Lösung (3,47 g) (19,2 mMol) zu. Man rührte 2 h bei 62 °C. Das Lösungsmittel wurde vollständig abdestilliert Nach NMR-Analyse erhielt man das 3-Methoxy-5-methyl-1-oxypyrazin-2-ylamin
   Ausbeute 90%
b) Unter Argon wurden das 3-Brom-5-methyl-1-oxopyrazin-2-ylamin (4,2 g) (20 mMol) Methanol (22,5 g), Wasser (21,2 g) und KOH (1,4 g) (21,2 mMol) in einem Kolben vorgelegt. Bei 70 °C rührte man 3,5 h. Nach der Destillation von Methanol extrahierte man das Produkt mit 20 ml n-Butanol (dreimal). Man erhielt 3,07 g 3-Methoxy-5-methyl-1-oxypyrazin-2-ylamin.
Ausbeute. 99%

| | |
|---|---|
| ¹H-NMR (DMSO-d₆, 400 MHz) δ· | 2,10 (s, 3H); |
| | 3,96 (s, 3H); |
| | 6,50 (s, 2H); |
| | 7,66 (s,1H). |

### Beispiel 5

### Herstellung von 3-Methoxy-5-methylpyrazin-2-amin

Das 3-Methoxy-5-methyl-l-oxopyrazin-2-ylamin (1 g) (6,44 mMol) und Methanol (50 ml) wurden mit 0,19 g Pt / C-5% im Autoklav vorgelegt. Man spülte zuerst mit Argon (dreimal) und dann presste man Wasserstoff auf 10 bar. Die Hydrierung lief 5 h bei 130 °C. Bei 20 °C spülte man mit Argon.Der Katalysator wurde filtriert und mit 5 ml Methanol gewaschen Das Lösungsmittel wurde vollständig abdestilliert. Man erhielt 0,85 g 3-Methoxy-5-methylpyrazin-2-amin
Ausbeute: 95%

| | |
|---|---|
| ¹H-NMR (DMSO-d₆, 400 MHz) δ | 2,20 (s, 3H); |
| | 3,87 (s, 3H); |
| | 5,90 (s, 2H); |
| | 7,33 (s, 1H) |

Schmelzpunkt 75 - 76,5 °C

### Beispiel 6

### Herstellung von 5-Methyl-1-oxy-pyrazin-2-amin

Isonitrosoaceton (18,7 g, 0,21 mol) und Methylmorpholin (22,0 g; 0,213 mol) wurden mit Chloroform (200 ml) in einem Kolben unter Argon vorgelegt. Man gab portionsweise Aminoacetonitrilhydrochlorid (24,35 g; 0,257 mol) bei 65 °C innerhalb 2 h hinzu und rührte dann noch 2 h bei dieser Temperatur. Anschliessend gab man bei 20 °C Wasser (80 ml) und HCI (32%ig; 12,9 g) hinzu. Die rohe Lösung wurde über Celit filtriert und dann wurden die Phasen getrennt Die Wasserphase wurde mit NaOH (30%ig; 71,0 g) auf pH 11 eingestellt. Das Produkt wurde mit 2-Methyl-2-butanol (4 mal je 60 ml) bei 40 °C extrahiert. Die organische Phase wurde vollständig abdestilliert Man erhielt 7,68 g des Produktes entsprechend einer Ausbeute von 30 %.

| | |
|---|---|
| NMR (DMSO-d₆) (400 Mhz) | 2,24 (s, 3H); |
| | 6,70 (s, 2H), |
| | 8,02 (s, 2H). |

### Beispiel 7

### Herstellung von 3-Methoxy-5-methyl-1-oxypyrazin-2-ylamin (ohne Isolation von 3-Brom-5-methyl-1-oxypyrazin-2-ylamin)

a) 5-Methyl-1-oxy-pyrazin-2-amin (2,0 g; 15,9 mMol), KOH (85%ig, 1,21 g, 18,3 mMol), Wasser (6 g), Methanol (9,5 g) wurden in einem Kolben unter Argon vorgelegt. Dann wurde innerhalb 30 min. bei 3 °C Brom (4,92 g; 30,7 mMol) zugetropft. Die Temperatur stieg bis auf 10 °C. Man rührte 1 h bei 20 °C und gab KOH (64 mmol) und Methanol (7,9 g) hinzu. Dann rührte man 2 h bei 72 °C. Nach Abdestillieren des Methanols extrahierte man das Produkt mit 2-Methyl-2-butanol (3 mal je 40 ml) Die organische Phase wurde vollständig eingeengt Nach dem Trocknen erhielt man 1,58 g des Produktes entsprechend einer Ausbeute von 63 %.

| | |
|---|---|
| NMR (DMSO-d₆) | 2,10 (s, 3H); |
| | 3,96 (s, 3H); |
| | 6,50 (s, 2H), |
| | 7,66 (s, 1H) |

b) Das 3-Amino-6-methyl-4-oxopyrazin-2-carbonitril (20 g) (129,8 mMol), KOH (13,36 g-85%ig) (203,9 mMol) und Wasser (130 g) wurden in einem Kolben unter Argon vorgelegt und 3 h bei 45 °C aufgewarmt Dann wurde bei 9 °C in 30 min 33,58 g Brom (210 mMol) und 25 g Methanol zugetropft (Exothermie und Gasentwicklung) Die Temperatur stieg bis 13 °C. Man rührte 1 h bei 20 °C und man gab 19,14 g KOH (290 mMol) und 63 g Methanol zu. Man rührte 2 h bei 72 °C. Nach der Methanol-Destillation extrahierte man das Produkt kontinuierlich mit 2-Methyl-2-butanol. Das 3-Methoxy-5-methyl-1-oxopyrazin-2-ylamin fiel in 2-Methyl-2-butanol aus Nach Filtration bei 5 °C und Trocknung, erhielt man 21,05 g von 3-Methoxy-5-methyl-1-oxopyrazin-2-ylamin. Ausbeute 82%

## Patentansprüche

1. Verfahren zur Herstellung von Pyrazin-2-aminen der allgemeinen Formel 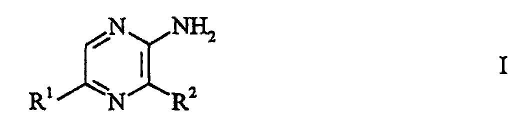 worin R¹ eine C₁₋₄-Alkylgruppe oder eine Arylgruppe und R² eine C₁₋₄-Alkoxygruppe oder eine Aryloxygruppe bedeutet, **dadurch gekennzeichnet, dass** man in der ersten Stufe Aminomalonsäuredinitril, oder dessen Salz, der Formel 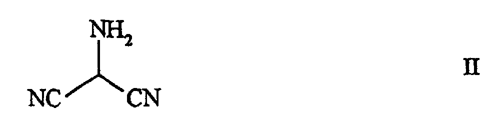 mit einem Glyoxaloximderivat der allgemeinen Formel 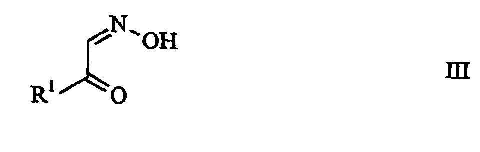 worin R¹ die genannte Bedeutung hat, auf an sich bekannte Weise in ein Oxypyrazincarbonitrilderivat der allgemeinen Formel 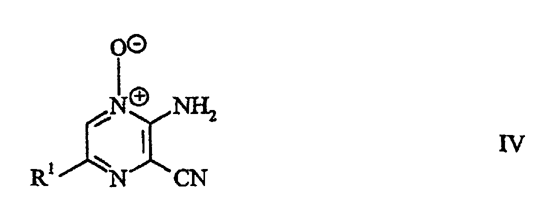 worin R¹ die genannte Bedeutung hat, überführt, dieses in der zweiten Stufe in ein Oxypyrazincarbonsäuresalz der allgemeinen Formel 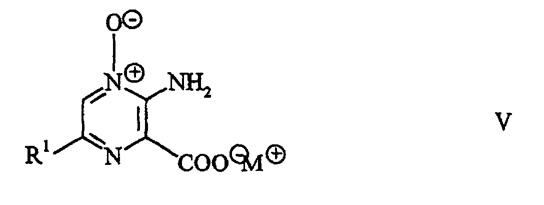 worin R¹ die genannte Bedeutung hat und M ein Metallatom oder Ammonium bedeutet, hydrolysiert, dieses in der dritten Stufe zu einem Halooxypyrazinamin der allgemeinen Formel 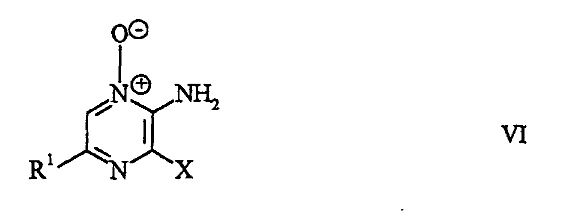 worin R¹ die genannte Bedeutung hat und X ein Halogenatom bedeutet, halogeniert, dieses in der vierten Stufe mit einem Alkohol in Gegenwart einer Base oder mit einem Alkoholat in ein Oxypyrazin-2-ylamin der allgemeinen Formel 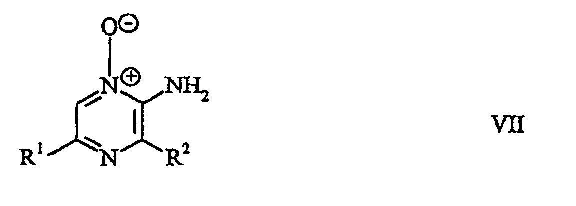 worin R¹ und R² die genannte Bedeutung haben, überführt und letzeres in der fünften Stufe ins Endprodukt der allgemeinen Formel I reduziert.

2. Verfahren nach Anspruch 1, **dadurch gekennzeichnet, dass** man die Hydrolyse in der zweiten Stufe in Gegenwart einer Base oder einer Säure durchführt.

3. Verfahren nach einem der Ansprüche 1 oder 2, **dadurch gekennzeichnet, dass** man in der zweiten und vierten Stufe als Base ein Alkalimetallhydroxid, ein Erdalkalimetallhydroxid oder ein Ammoniumsalz verwendet.

4. Verfahren nach mindestens einem der Ansprüche 1 bis 3, **dadurch gekennzeichnet, dass** man als Alkoholat in der vierten Stufe ein C₁₋₄-Alkalimetallaloholat oder ein Arylalkalimetallalkoholat verwendet.

5. Verfahren nach mindestens einem der Ansprüche 1 bis 4, **dadurch gekennzeichnet, dass** man die Reduktion in der fünften Stufe katalytisch mit Wasserstoff, PCl₃ oder Na₂S₂O₄ durchführt.

6. Verfahren nach Anspruch 5, **dadurch gekennzeichnet, dass** man als Hydrierkatalysator in der vierten Stufe Platin auf Kohle verwendet.

7. Verfahren nach mindestens einem der Ansprüche 1 bis 6, **dadurch gekennzeichnet, dass** man die Umsetzung ohne Isolation des Zwischenproduktes der Formel V durchführt.

8. Verfahren zur Herstellung von Pyrazin-2-aminen der allgemeinen Formel 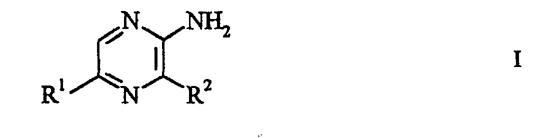 worin R¹ und R² die in Anspruch 1 genannte Bedeutung haben, **dadurch gekennzeichnet, dass** man in der ersten Stufe Aminoacetonitril, oder dessen Salz, der Formel
H₂N-CH₂-CN VIII
mit einem Glyoxaloximderivat der allgemeinen Formel 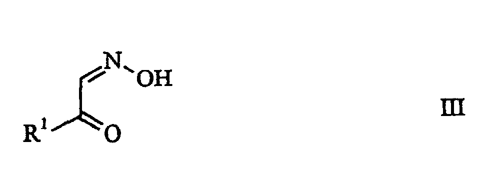 worin R¹ die genannte Bedeutung hat, in Gegenwart einer Base in ein Oxypyrazinamin der allgemeinen Formel 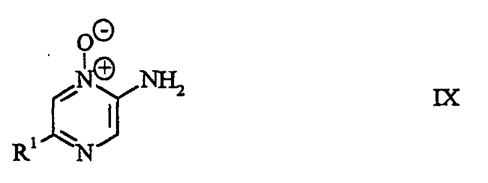 worin R¹ die genannte Bedeutung hat, überführt, dieses in der zweiten Stufe zu einem Halooxypyrazinamin der allgemeinen Formel 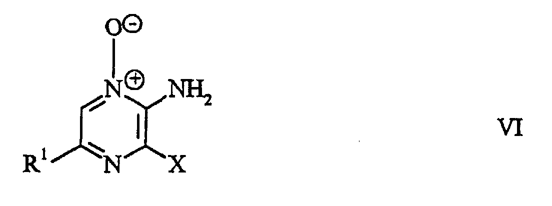 worin R¹ und X die genannte Bedeutung haben, halogeniert, dieses in der dritten Stufe mit einem Alkohol in Gegenwart einer Base oder mit einem Alkoholat in ein Oxypyrazin-2-ylamin der allgemeinen Formel 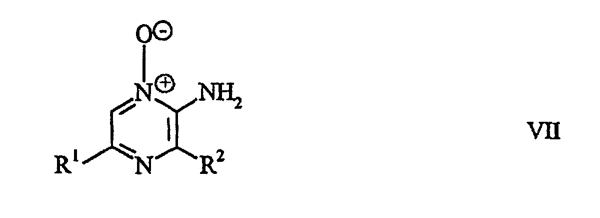 worin R¹ und R² die genannte Bedeutung haben, überführt und letzeres in der vierten Stufe ins Endprodukt der allgemeinen Formel I reduziert.

9. Verfahren nach Anspruch 8, **dadurch gekennzeichnet, dass** man die Umsetzung ohne Isolation des Zwischenproduktes der Formel VI durchführt.

10. Oxypyrazincarbonsäuresalze der allgemeinen Forme 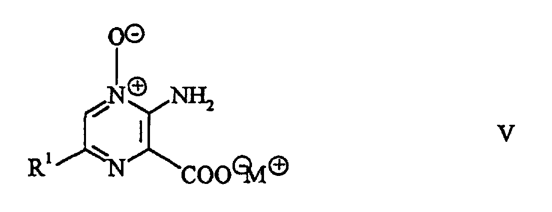 worin R¹ und M die in Anspruch 1 genannte Bedeutung haben.

11. Halooxypyrazinamine der allgemeinen Formel 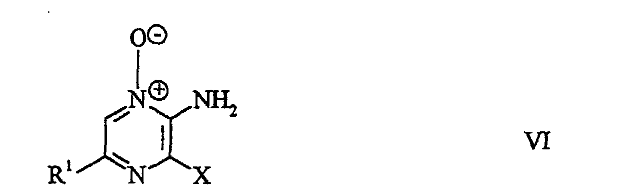 worin R¹ und X die in Anspruch 1 genannte Bedeutung haben.

12. Oxypyrazin-2-ylamine der allgemeinen Formel 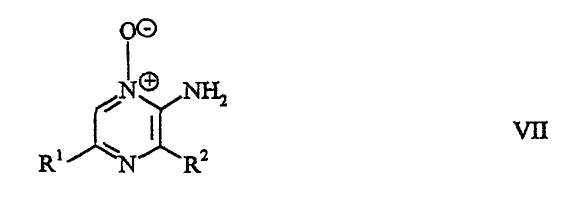 worin R¹ und R² die in Anspruch 1 genannte Bedeutung haben.

## Claims

1. Method of producing 2-amino-pyrazines having the general formula 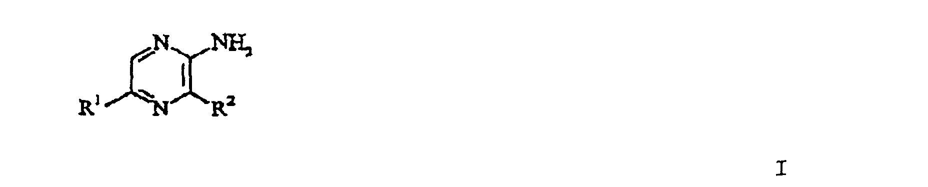 in which R¹ stands for a C₁₋₄ alkyl group or an aryl group and R² stands for a C₁₋₄ alkoxy group or an aryloxy group, **characterised in that**, in the first step amino-malonic acid nitrile or a salt thereof having formula 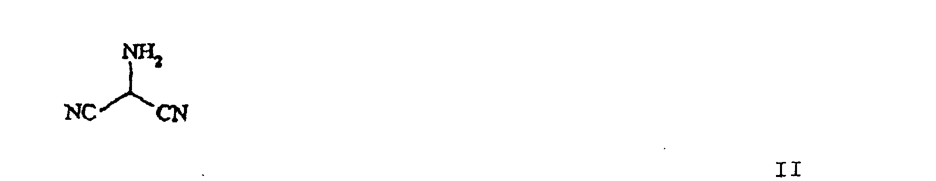 is converted in a known manner with a glyoxaloxime derivative having the general formula 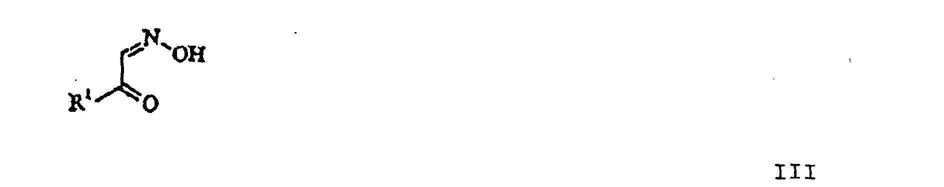 in which R¹ has said meaning, into an oxypyrazine-carbonitrile derivative having the general formula 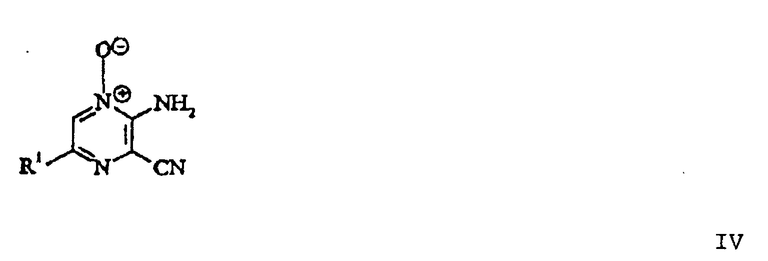 in which R¹ has said meaning, in the second step the latter is hydrogenated to obtain an oxypyrazine-carboxylic acid salt having the general formula 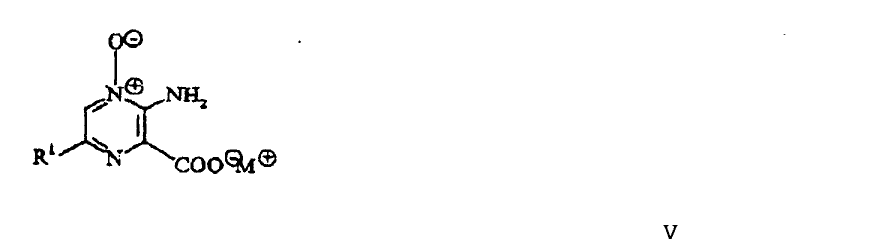 in which R¹ has said meaning and M stands for a metal atom or ammonium, in the third step the latter is halogenated to obtain a halo-oxy-amino-pyrazine having the general formula 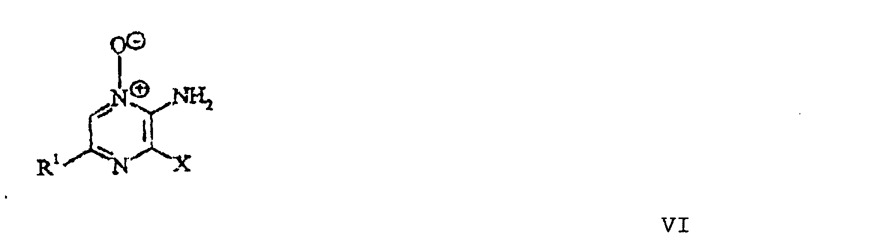 in which R¹ has said meaning and X stands for a halogen atom, in the fourth step the latter is converted with an alcohol in the presence of a base or with an alcoholate into an oxy-amino-pyrazin-2-yl having the general formula 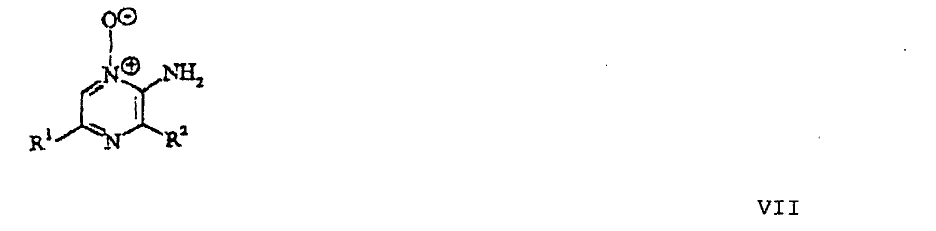 in which R¹ and R² have said meaning, and in the fifth step the latter is reduced to the end product having the general formula I.

2. Method as claimed in claim 1, **characterised in that** the hydrolysis in the second stage is effected in the presence of a base or an acid.

3. Method as claimed in one of claims 1 or 2, **characterised in that** an alkali metal hydroxide, an earth alkali metal hydroxide or an ammonium salt is used as a base in the second and fourth stage.

4. Method as claimed in one of claims 1 to 3, **characterised in that** a C₁₋₄-alkali metal alcoholate or an aryl alkali metal alcoholate is used as the alcoholate in the fourth step.

5. Method as claimed in at least one of claims 1 to 4, **characterised in that** the reduction in the fifth stage is effected by catalysis with hydrogen, PCl₃ or Na₂S₂O₄.

6. Method as claimed in claim 5, **characterised in that** platinum on charcoal is used as the hydrogenation catalyst in the fourth step.

7. Method as claimed in at least one of claims 1 to 6, **characterised in that** the conversion takes place without isolating the intermediate product having formula V.

8. Method of producing 2-amino-pyrazines having the general formula 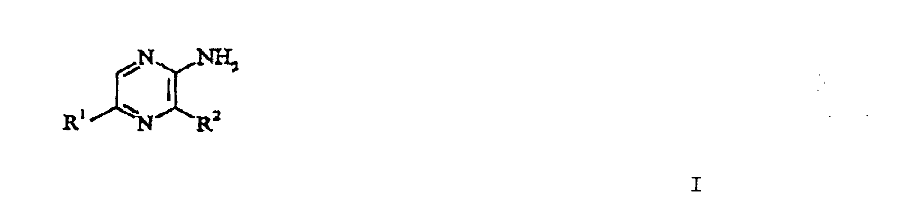 in which R¹ and R² have the meaning specified in claim 1, **characterised in that** in the first step amino-acetonitrile or a salt thereof having formula
H₂N-CH₂-CH VIII
is converted with a glyoxaloxime derivative having the general formula 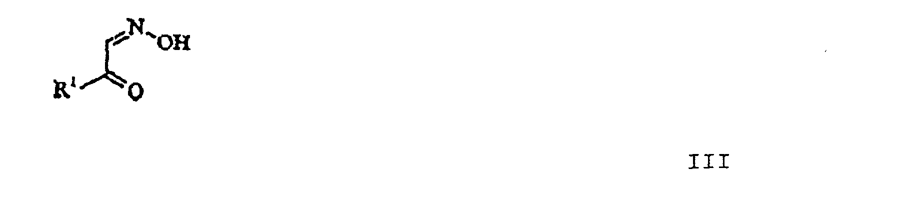 in which R¹ has said meaning, in the presence of a base, into an oxy-amino-pyrazine having the general formula 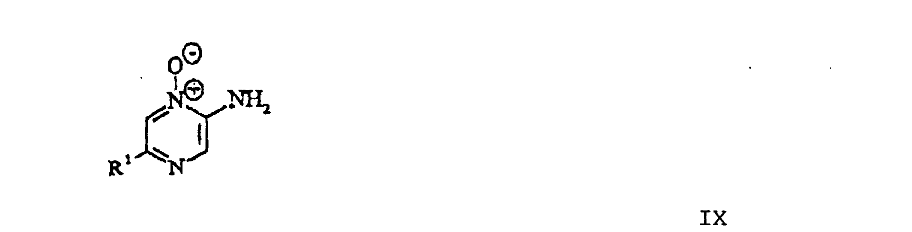 in which R¹ has said meaning, in the second stage the latter is halogenated into a halo-oxy-amino-pyrazine having the general formula 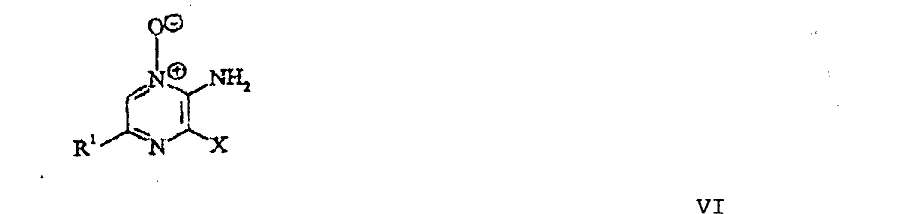 in which R¹ and X have said meaning, in the third stage the latter is converted with an alcohol in the presence of a base or with an alcoholate into an oxy-amino-pyrazin-2-yl having the general formula 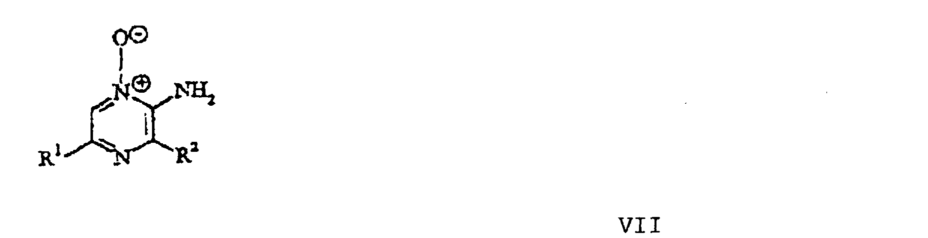 in which R¹ and R² have said meaning and in the fourth stage the latter is reduced to the end product having the general formula I.

9. Method as claimed in claim 8, **characterised in that** the conversion is effected without isolating the intermediate product having formula VI.

10. Oxy-pyrazine carboxylic acid salt having the general formula 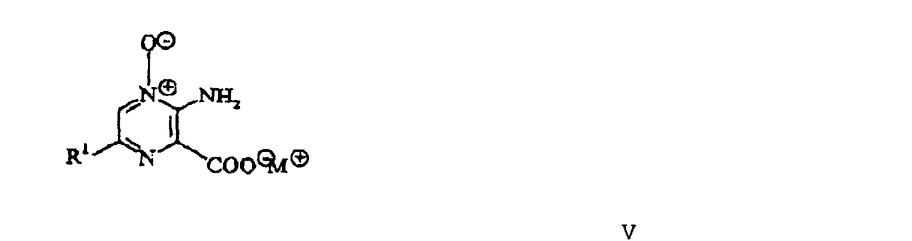 in which R¹ and M have the meaning specified in claim 1.

11. Halo-oxy-amino-pyrazine having the general formula 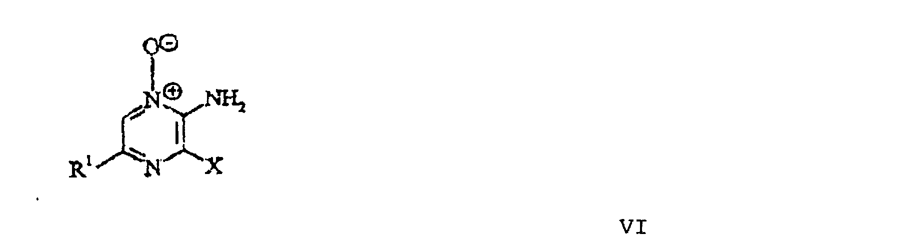 in which R¹ and X have the meaning specified in claim 1.

12. Oxy-amino-pyrazin-2-yl having the general formula 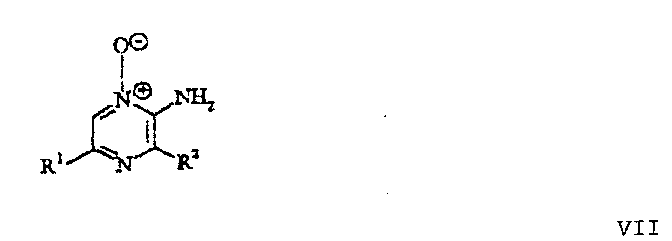 in which R¹ and R² have the meaning specified in claim 1.

## Revendications

1. Procédé de préparation de pyrazin-2-amines de la formule générale 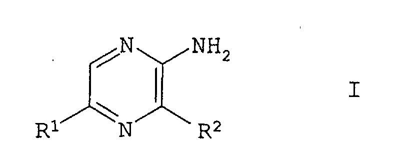 dans laquelle R¹ représente un groupe alkyle en C₁ à C₄ ou un groupe aryle et R² représente un groupe alcoxy en C₁ à C₄ ou un groupe aryloxy, **caractérisé en ce que**, dans la première étape, on convertit un dinitrile d'acide amino-malonique, ou son sel, de la formule 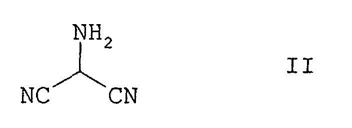 avec un dérivé d'oxime de glyoxal de la formule générale 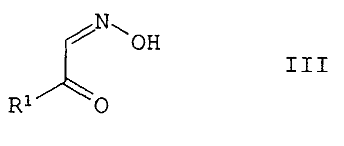 dans laquelle R¹ a la signification indiquée, d'une manière en soi connue, en un dérivé de carbonitrile d'oxypyrazine de la formule générale 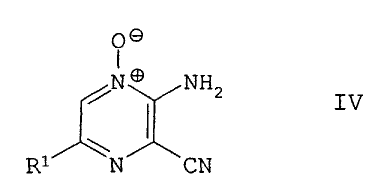 dans laquelle R¹ a la signification indiquée ; **en ce que**, dans la deuxième étape, on hydrolyse ce dernier en un sel d'acide oxypyrazinecarboxylique de la formule générale 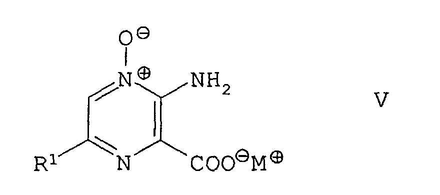 dans laquelle R¹ a la signification indiquée et M représente un atome de métal ou un ammonium ; **en ce que**, dans la troisième étape, on halogène ce dernier en une halo-oxypyrazinamine de la formule générale 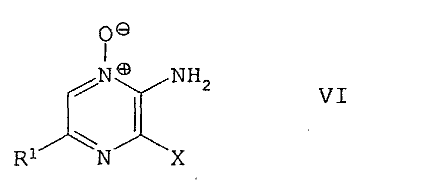 dans laquelle R¹ a la signification indiquée et X représente un atome d'halogène ; **en ce que**, dans la quatrième étape, on convertit cette dernière, avec un alcool en présence d'une base ou. avec un alcoolate, en une oxypyrazin-2-ylamine de la formule générale 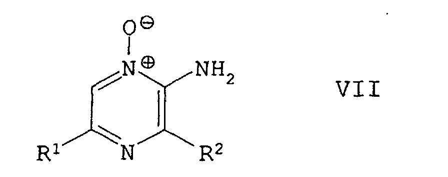 dans laquelle R¹ et R² ont la signification indiquée ; et **en ce que**, dans la cinquième étape, on réduit cette dernière en produit final de la formule I.

2. Procédé selon la revendication 1, **caractérisé en ce que**, dans la deuxième étape, on effectue l'hydrolyse en présence d'une base ou d'un acide.

3. Procédé selon l'une des revendications 1 ou 2, **caractérisé en ce que**, dans la deuxième et dans la quatrième étape, on utilise comme base un hydroxyde de métal alcalin, un hydroxyde de métal alcalino-terreux ou un sel d'ammonium.

4. Procédé selon l'une au moins des revendications 1 à 3, **caractérisé en ce que**, dans la quatrième étape, on utilise comme alcoolate un alcoolate en C₁ à C₄ de métal alcalin ou un arylalcoolate de métal alcalino-terreux.

5. Procédé selon l'une au moins des revendications 1 à 4, **caractérisé en ce que**, dans la cinquième étape, on effectue la réduction de manière catalytique avec de l'hydrogène, du PCl₃ ou du Na₂S₂O₄.

6. Procédé selon la revendication 5, **caractérisé en ce que**, dans la quatrième étape, on utilise comme catalyseur d'hydrogénation du platine sur charbon.

7. Procédé selon l'une au moins des revendications 1 à 6, **caractérisé en ce que** l'on effectue la conversion sans isoler le produit intermédiaire de la formule V.

8. Procédé de préparation de pyrazin-2-amines de la formule générale 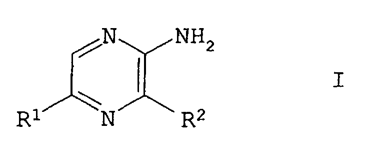 dans laquelle R¹ et R² ont la signification indiquée, **caractérisé en ce que**, dans la première étape, on convertit un aminoacétonitrile, ou son sel, de la formule
H₂N-CH₂-CN VIII
avec un dérivé d'oxime de glyoxal de la formule générale 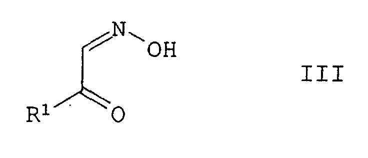 dans laquelle R¹ a la signification indiquée, en présence d'une base, en une oxypyrazinamine de la formule générale 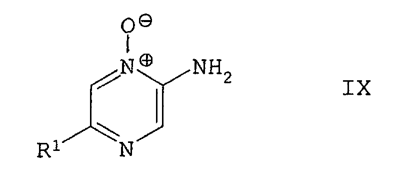 dans laquelle R¹ a la signification indiquée ; **en ce que**, dans la deuxième étape, on halogène cette dernière en une halo-oxypyrazinamine de la formule générale 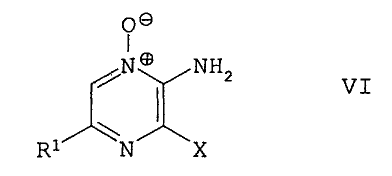 dans laquelle R¹ et X ont la signification indiquée ; **en ce que**, dans la troisième étape, on convertit cette dernière, avec un alcool en présence d'une base ou avec un alcoolate, en une oxypyrazin-2-ylamine de la formule générale 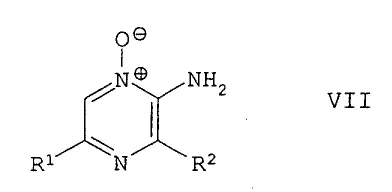 dans laquelle R¹ et R² ont la signification indiquée ; et **en ce que**, dans la quatrième étape, on réduit cette dernière en produit final de la formule I.

9. Procédé selon la revendication 8, **caractérisé en ce que** l'on effectue la conversion sans isoler le produit intermédiaire de la formule VI.

10. Sels d'acide oxypyrazinecarboxylique de la formule générale 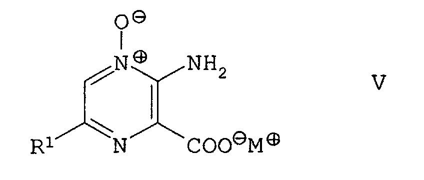 dans laquelle R¹ et M ont la signification indiquée dans la revendication 1.

11. Halo-oxypyrazinamines de la formule générale 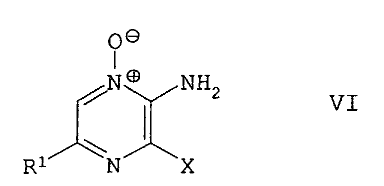 dans laquelle R¹ et X ont la signification indiquée dans la revendication 1.

12. Oxypyrazin-2-ylamines de la formule générale 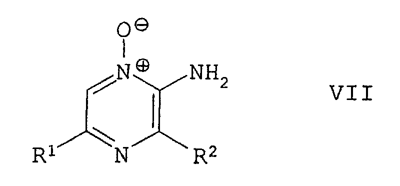 dans laquelle R¹ et R² ont la signification indiquée dans la revendication 1.
